# EUROPEAN PATENT SPECIFICATION

(11) **EP 3 426 202 B1**
(45) Date of publication and mention of the grant of the patent: **15.04.2026**
(21) Application number: 17708803.6
(22) Date of filing: 06.03.2017
(51) Int. Cl.: A61F 5/44, A61F 5/445

(54) **DIAGNOSTIC UROSTOMY BAG**
DIAGNOSTIKUROSTOMIEBEUTEL
POCHE D'UROSTOMIE DIAGNOSTIQUE

(30) Priority: 11.03.2016 GB 201604175
(43) Date of publication of application: 16.01.2019
(73) Proprietor: WELLAND MEDICAL LIMITED, Crawley West Sussex RH10 9AS (GB)
(72) Inventor: MOAVENIAN, Arash, Crawley West Sussex RH10 9AS (GB)
(74) Representative: Lock, Graham James
(86) International application number: PCT/EP2017/055163
(87) International publication number: WO 2017/153320

(56) References cited:
- EP-A1- 1 090 613
- WO-A1-90/03771
- WO-A2-2007/076445
- DE-A1- 3 504 527
- GB-A- 2 186 079
- GB-A- 2 487 223
- GB-A- 2 528 305

## Description

### FIELD OF THE INVENTION

This invention relates to a diagnostic urostomy bag. The invention also relates to a method of producing the diagnostic urostomy bag. Document EP 1 090 613 A1 discloses the features of the preamble of claim 1.

### BACKGROUND OF THE INVENTION

The urinary system comprises the kidneys, ureters, bladder and urethra and serves to remove waste from the body in the form of urine. The kidneys filter waste from blood and produce urine which is carried to the bladder through the ureters, with the bladder serving as a storage reservoir for the urine before it exits the body through the urethra. Urinary tract infections (UTIs) most commonly occur in the lower tract, infecting the urethra (urethritis) and bladder (cystitis) but can also affect the ureters (ureteritis) and kidneys (pyelonephritis). UTIs mainly arise due to bacteria (commonly *Escherichia coli*) but may also be attributed to viruses or fungi.

Urinary tract infections (UTIs) are the second most common type of infection in the body, accounting for 1-3% of all general practice consultations, with a high rate of recurrences, in particular in women. Healthcare associated UTIs are mainly related to catheterisation and it is estimated (NICE quality standard 61) that UTIs make up 17.2% (51,600 patients) of the 300,000 patients in England acquiring a health-care associated infection.

UTIs present symptoms including: frequent and intense urge to urinate, painful burning sensation in the bladder or urethra during urination, muscle aches, abdominal pain, urine with a dark, cloudy appearance, presence of blood and foul smell in urine and fever. Aetiologies can include: spinal cord injury or other nerve damage around the bladder preventing complete emptying of the bladder and where chronic use of catheterisation is necessary, abnormalities in the urinary tract obstructing the flow of urine (e.g. kidney stones), diabetes, sexual activity (birth control, spermicides, diaphragms and condoms), use of catheters in the urethra or bladder and pregnancy (4-5% develop a UTI), which can lead to shifts in position of the urinary tract. Other risk factors include diabetes, being uncircumcised and an enlarged prostate as well as a genetic predisposition.

Women are most commonly affected, with an estimated 50% of all women in the UK at some point in their lifetime developing a UTI. The reason for this is the shorter urethra in women which opens in closer proximity to the anus, increasing the risk of bacteria entering the urinary system and causing infection. Also vaginal atrophy and declining oestrogen levels post-menopause, which results in loss of the protective vaginal flora, can increase the risk of infection. The prostate gland in men produces an antibacterial component which further reduces the risk of infection. Symptoms are more difficult to diagnose in elderly patients, who may have pre-existing incontinence. In this group patients can present in the first instance with sepsis - an infection of the blood.

Diagnoses of UTIs can be made based on either or both symptoms and urinalysis. UTIs present symptoms including: frequent and intense urge to urinate, painful burning sensation in the bladder or urethra during urination, muscle aches, abdominal pain, urine with a dark, cloudy appearance, presence of blood and foul smell in urine and fever. Urinalysis identifies the presence of urinary nitrites and white blood cells (leukocytes), where dipstick test kits can be used at home, without prescription; since bacteria can be found in healthy individuals, tests need to be read alongside symptoms. Urine microscopy may also be used to identify bacteria. MRI, CT and ultrasound scans can also be used to observe any abnormality in the urinary system.

Treatment usually involves antibiotics, either a short term course or in low doses as a preventative measure in those experiencing recurrences. In more complicated cases, urine culture may also be used where urine samples are kept in conditions under which bacteria can grow to test positivity. The type of organism causing the infection can then be confirmed through microscopy or chemical tests and sensitivity testing used to identify the most suitable form of antibiotic.

Urostomy bags are medical devices that are worn by urostomates and they can be used for the collection of waste from a surgically diverted urinary system of the individual. They are used to collect waste that is output from a stoma created in the ostomate's skin and connected to the urinary system.

Known urostomy bags comprise a collection pouch and in some cases the collection pouch is attached mechanically or with adhesive to a flange, commonly referred to as a mounting plate, wafer or a baseplate. The flange is fixed to the skin of an individual and the urostomy bag allows waste to drain from a stoma into the collection pouch, while protecting the surrounding skin from contamination by the waste.

Urostomy bags should be air- and water-tight and they should allow the urostomate to lead an active normal lifestyle that can include all forms of sports and recreation.

Urostomates are more prone to UTIs than those who do not have an ostomy, mainly due to an abnormal urinary tract. Surgery in this patient group can lead to loss of valves normally present at the ends of the ureters, which would otherwise serve to prevent the backflow of urine. Bacteria are therefore more likely to end up directly at the kidneys, resulting in a kidney infection. This along with problematic fitting, use of urostomy bags without non-return valves with some patients and excess accumulation of urine in the urostomy bag can further increase the risk of infection.

While the great majority of UTIs present as lower tract infections of the bladder and urethra, ostomates present with infections of the kidney. The type of antibiotic usually prescribed is for lower urinary tract infections and is therefore not suitable for the ostomy patient.

Progressive infection of the kidneys if not treated can be fatal. Without treatment temperatures can continue to rise resulting in unconsciousness and patients often have to be hospitalised for intravenous administration of antibiotics. Thus, there is a need to make early urinary tract infection easy to detect for the urostomate population.

Current methods of detection for UTIs utilise non-prescriptive home kit test strips. The limitation of this in the urostomate population is the incapacity to urinate on demand and having to deal with yet another device in addition to the bag and accessories, which renders monitoring difficult and may reduce uptake.

Urostomy patients not only suffer from the complications surrounding stoma formation but are a population in which urinary tract infections (UTIs) are prevalent and in a large number of cases recurring.

Accordingly, there is a need for development of an integrated diagnostic urostomy bag system that acts effectively.

The present invention seeks to provide an alternative or improvement which preferably addresses one or more of the problems presented by prior art arrangements.

### SUMMARY OF THE INVENTION

The present invention is directed to providing an ostomy bag having an integrated diagnostic device which allows early detection of urinary tract infection in urostomates.

Remarkably, this allows production of an ostomy bag with technical advantages as will be detailed below.

In accordance with a first aspect, the present invention provides an ostomy bag for an ostomate which comprises a flange adjoining at least one pouch wherein the pouch has an integrated diagnostic device for detection of urinary tract infection and an inlet leading to a waste collection space defined by two opposing surfaces of two walls, the opposing surfaces configured to separate on the introduction of waste through the inlet.

Preferably, the ostomy bag is a urostomy bag.

Advantageously, the invention provides an easy-to-use and compliance-friendly approach to monitoring urine content.

Preferably, the diagnostic device is provided in at least one wall of the pouch. Preferably, in use, it is provided in a wall facing away from the ostomate. Preferably, it is provided in the front side of the pouch facing away from the user. This provides the advantage that the ostomate can easily inspect the diagnostic device. Preferably, the flange adjoins a first wall of the pouch and the diagnostic device adjoins a second wall of the pouch.

Preferably, the diagnostic device comprises a detection cell.

Preferably, the diagnostic device is located substantially at or adjacent the bottom of the pouch in use. In this regard, preferably the pouch is drainable and the diagnostic device is positioned at or adjacent a drain outlet from the pouch where it is likely to come into complete contact with an ostomate's urine. This has the advantage of maximising contact with urine when urine is located in the pouch. In this regard, the detection cell of the diagnostic device is located so that a volume of about 100ml of urine in the pouch substantially wets the detection cell. Preferably, in use, the fluid level of a volume of about 100ml waste in the bag is above the level of the detection cell.

Preferably, the detection cell detects one or more substances in the waste and indicates information regarding one or more of a user's level of hydration, the presence of blood or other disease or condition, including type I diabetes.

Preferably, the diagnostic device comprises an indicator panel.

Preferably the indicator panel comprises a first indicator, which provides an optical signal when the detection cell comes into contact with moisture. Preferably, the optical signal is a change of colour. Preferably, the indicator comprises a web of material coated in an absorbent material and a chemical or reagent which changes colour on contact with moisture. Advantageously, this provides the user with a clear indication of whether urine has contacted the detection cell. Preferably, the web of material is rectangular, has a width of at least about 5mm and a length of at least about 8mm, but it can be of any appropriate size and shape as long as it is of sufficient size to be seen clearly by the user.

Preferably, the indicator panel comprises at least one further indicator, preferably adjacent to the first indicator, which provides an optical signal, after contact with waste in the ostomy bag. Preferably, the optical signal is no change or a change of colour. Preferably, the indicator comprises a web of material coated in an absorbent material and a chemical or reagent which changes colour on contact with leukocyte esterase or a nitrite thereby indicating the presence in the waste of leukocytes or white blood cells and/or a nitrite to give an indication of presence of a UTI. More preferably, the indicator is capable of detecting and indicating concentrations in the waste of leukocytes or white blood cells and/or a nitrite to give an indication of presence of a UTI.

Preferably, the indicator panel comprises at least three indicators. Preferably, they are arranged adjacent to each other. A first indicator provides an optical signal when the detection cell comes into contact with fluid; a second indicator is capable of detecting and indicating the presence in the waste of leukocytes or white blood cells; and a third indicator is capable of detecting and indicating the presence in the waste of a nitrite.

Preferably, the diagnostic device comprises a laminated structure having a number of layers.

An inner layer of the diagnostic device preferably comprises a planar member preferably of plastics material. Preferably, the planar member has a width of about 2cm and a length of about 2cm. Preferably, the planar member defines an opening in its approximate centre, through which any urine in the pouch is able to pass. In use, urine from a user collects in the pouch and enters the detection cell of the diagnostic device via the opening.

Preferably, a sheet is attached to the side of the planar member facing outwardly from the pouch and a recess is provided over the opening in the planar member. Preferably, the sheet is of plastics material. Preferably, it is transparent and more preferably it is colourless.

The recess preferably contains a web of plastics material and on the web is provided the indicator panel of the detection cell. In an embodiment, the indicator panel comprises a nitrite detection indicator which comprises an acid which reacts with nitrite. A further reaction involving an amine produces a colourful azo compound. In addition, in an embodiment, the indicator panel comprises a leukocyte esterase indicator which involves the use of an ester and the formation of azo compound from a diazonium salt.

The indicator panel comprises indicators which preferably comprise pieces of absorbent material and which preferably provide an optical signal when a reagent on the absorbent material reacts with a specific analyte of interest. For example, a colour change of one or more indicators can signify the presence of an infection.

Preferably, urine from a user flows onto the indicator panel by the use of an absorbent material.

Preferably an outer layer of the detection cell is provided by a colourless film, which is impermeable to urine and forms the outer wall of the pouch over the detection cell.

Preferably, the detection cell is incorporated into an ostomy bag during the production assembly line. In this regard, the detection cell is preferably welded to one or more of the ostomy bag liners. Preferably, the welding is around the periphery of the detection cell. Alternatively the device may be glued to one or more parts of the ostomy bag.

The detection cell may be visible externally or preferably a window is present in the outer pouch, allowing the detection cell to be viewed upon manipulation or movement of an outer softback layer.

With an ever increasing trend towards patient empowerment, the invention has the advantage of reducing patient anxiety and apprehension associated with having an undiagnosed and deteriorating state of urinary tract infection; it provides the opportunity for self-monitoring, saving time and money and can be used for diagnostics even by users that have limited dexterity. Furthermore, the invention provides an opportunity for urostomates to utilise their bag in becoming more informed on their health and puts them at an advantage compared to the wider population.

The invention also provides advantages to the healthcare system because community care visits and the associated cost can be reduced, treatment time and cost associated with early detection and prevention of care required for treatment of advanced infection can be reduced, the need for separate prescriptive home test kits for UTIs is reduced; and the integrated system of the invention improves user compliance.

According to the invention, the pouch can be a closed pouch, or a drainable pouch having an opening through which the pouch may be drained.

In use, it is intended that urostomates could use an ostomy bag according to the invention periodically rather than on every pouch change. For example, an ostomy bag according to the invention could be used once per week, fortnightly, monthly, or periodically as required. In this regard, preferably, a pack of ostomy bags includes at least one ostomy bag according to the invention together with a number of known ostomy bags. Preferably, the ostomy bag of the invention is sealed in a package and identified as comprising a diagnostic device.

In accordance with a second aspect, the present invention provides a method for producing an ostomy bag according to the invention which comprises the steps of providing an integrated diagnostic device for detection of urinary tract infection in at least one wall of a pouch of an ostomy bag.

Preferably, the ostomy bag is a urostomy bag.

In a further aspect, the invention provides a method for monitoring or diagnosis of urinary tract infection in a urostomate which comprises the steps of periodically applying a ostomy bag according to the invention to a stoma of the urostomate and checking the integrated diagnostic device for any indication of urinary tract infection.

Preferably, the method comprises the step of applying an ostomy bag according to the invention periodically. Preferably this is once per week, fortnightly, monthly, or periodically as required.

### BRIEF DESCRIPTION OF THE DRAWINGS

The invention will now be further described with reference to the accompanying drawings in which:
Figure 1 shows an illustration of a diagnostic ostomy bag according to the invention (left) and an example of an integrated diagnostic device;
Figure 2 shows front and back views of a diagnostic urostomy bag according to the invention;
Figure 3 shows a typical care pathway for treatment of urinary tract infection with (top) and without (bottom) self-monitoring;
Figure 4 shows test strip results for Surine^{™} and the Surine^{™} with added nitrite and esterase;
Figure 5 shows a diagnostic device of an embodiment of the invention showing a positive result for the introduction of simulated infected urine; and
Figure 6 shows representations of diagnostic devices showing the colour transformations that took place in an embodiment of the invention. A pre-test diagnostic device is shown on the left and a post-test diagnostic device is shown on the right.

### DETAILED DESCRIPTION OF THE INVENTION

It will be appreciated that aspects, embodiments and preferred features of the invention have been described herein in a way that allows the specification to be written in a clear and concise way. However, unless circumstances clearly dictate otherwise, aspects, embodiments and preferred features can be variously combined or separated in accordance with the invention. Thus, preferably, the invention provides a device having features of a combination of two or more, three or more, or four or more of the aspects described herein. In a preferred embodiment, a device in accordance with the invention comprises all aspects of the invention.

The following definitions shall apply throughout the specification and the appended claims.

Within the context of this specification, the word "about" means preferably plus or minus 20%, more preferably plus or minus 10%, even more preferably plus or minus 5%, most preferably plus or minus 2%.

Within the context of this specification, the word "comprises" means "includes, among other things" and should not be construed to mean "consists of only".

Within the context of this specification, the word "substantially" means preferably at least 90%, more preferably 95%, even more preferably 98%, most preferably 99%.

Within the context of this specification, the word "urine" is used interchangably with the word "waste". It refers to a liquid by-product secreted by kidneys through a process called urination (or micturition) and excreted by a urostomate into a urostomy bag.

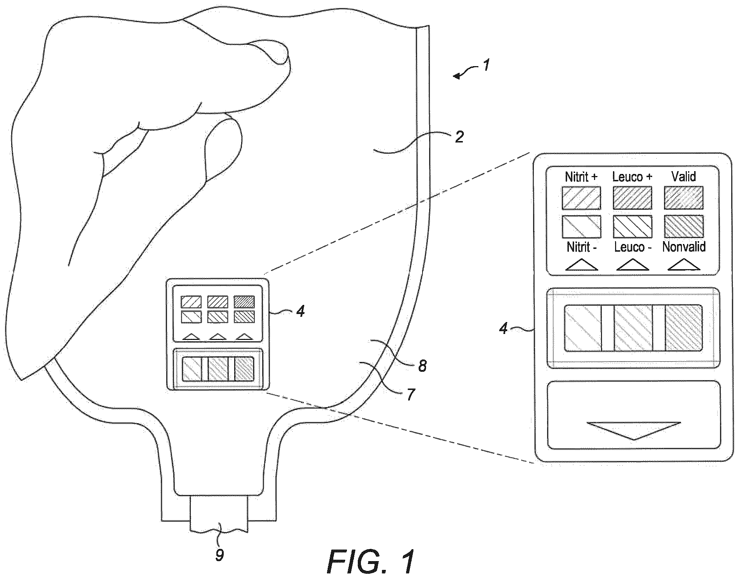

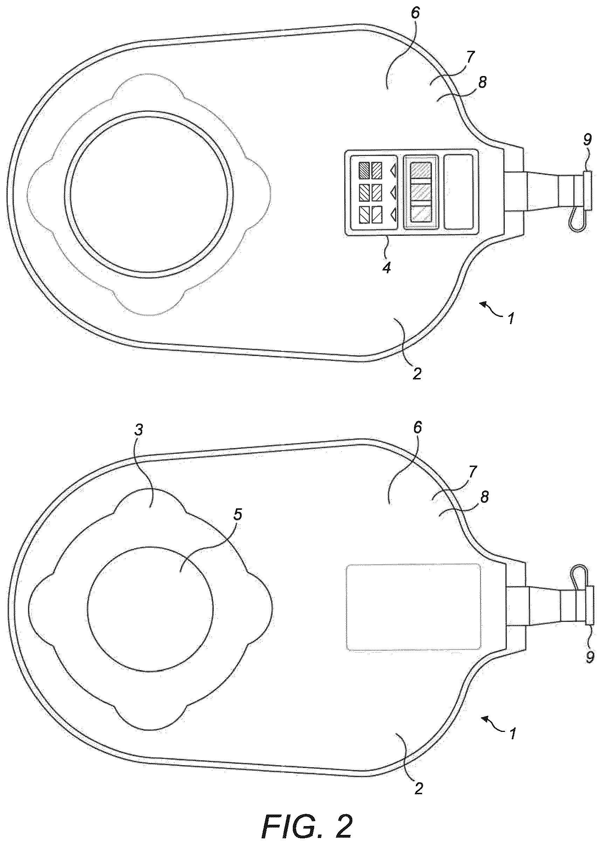

An ostomy bag (1) in accordance with the invention is shown in figures 1 and 2.

A diagnostic ostomy bag is a urostomy bag (1) which comprises a flange (3) adjoining at least one pouch (2) wherein the flange (3) is for abutment to the skin of an urostomate and the pouch (2) has an integrated diagnostic device (4) for detection of urinary tract infection and an inlet (5) leading to a waste collection space (6) defined by two opposing surfaces of two walls (7) (8), the opposing surfaces configured to separate on the introduction of waste through the inlet (5).

The diagnostic device (4) is provided in at least one wall (7) (8) of the pouch (2).

The diagnostic device (4) is located substantially at the bottom of the pouch (2) in use.

The diagnostic device (4) is capable of detecting and indicating concentrations of leukocytes or white blood cells and/or nitrites to give an indication of presence of a UTI.

The diagnostic device (4) detects and indicates additional information regarding one or more of a user's level of hydration, the presence of blood or other disease or condition including type I diabetes.

The pouch (2) is a drainable pouch having an opening (9) through which the pouch (2) may be drained.

A method for producing an ostomy bag (1) according to an embodiment of the invention comprises the steps of providing an integrated diagnostic device (4) for detection of urinary tract infection in at least one wall (7) (8) of a pouch (2) of an ostomy bag (1).

A method for monitoring or diagnosis of urinary tract infection in a urostomate comprises the steps of periodically applying a ostomy bag (1) according to the invention to a stoma of the urostomate and checking the integrated diagnostic device (4) for any indication of urinary tract infection.

The method comprises the step of applying an ostomy bag (1) according to the invention at least once per week or at least once per month.

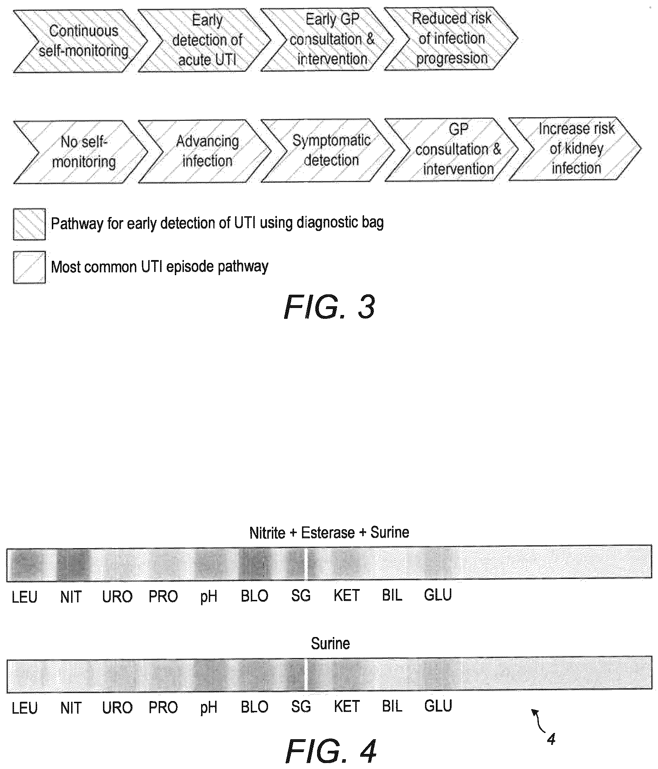

As shown in Figure 3, a typical care pathway for treatment of UTI includes a number of steps.

The top stream represents a typical pathway for early detection using a diagnostic home device. (Home kits are able to indicate presence of an UTI but cannot give information on its location in the urinary system). In this case, due to continuous self-monitoring, there is most probably a lower risk of infection progression which would require urine culture, microscopy and spread of infection to the kidneys. There will also be a major cost saving in prevention of any progression in infection.

The bottom stream represents a typical pathway where no self-monitoring takes place. Undetected UTI is most likely to result in the patient presenting symptoms of a more progressed infection and may require a stronger course of antibiotics and potential increase in hospitalisation time.

If bacteria progresses to the upper urinary tract this can lead to kidney infections. Chronic kidney infection can cause scarring, poor function, high blood pressure and acute kidney infection can be life threatening especially if the bacteria enter the blood stream.

It is clear that there are advantages provided by continuous self-monitoring, as can be carried out using a urostomy bag according to the invention.

### EXAMPLES

### Example 1

Testing Urine test strip in the presence of hydrocolloid

### Introduction

A test was created to ensure that the hydrocolloid does not produce any 'false positive' results when testing solutions exposed to hydrocolloid as the hydrocolloid contains an ingredient comprising protein. The Leukocyte and Nitrite tests are of particular interest used in the detection of UTI's. The presence of protein could affect the protein test and more importantly inhibit the Leukocyte test.

### Materials Used

Mission, Urinalysis Reagent Strips (ACON Laboratories, Inc.)
Phosphate buffered saline tablets (SIGMA Life Science).
Hydrocolloid (Welland Medical Limited)

### Test Method

A solution of phosphate buffered saline was prepared using PBS tablets dissolved in deionized water. This gives a solution of 0.01M phosphate buffer, 0.0027M potassium chloride and 0.137M sodium chloride, pH 7.4 at 25°C. This solution is used as a standard to simulate body fluids in terms of osmolarity.

Pieces of hydrocolloid were cut to give 0.1g/L, 0.5g/L, 1g/L, 10g/L and 93.4g/L which was the largest piece that would fit in a 100ml beaker and represents an excessive amount. The pieces of hydrocolloid were immersed in their relevant quantities of PBS and left for 1 hour. The hydrocolloid does not dissolve in this time period but swells significantly. This time period is considered comparable with that taken for an urostomate to produce enough urine to activate the test strip.

### Results from Hydrocolloid test

The test solutions were prepared as follows.
0.05g HC → 500ml PBS = 0.1g/L
0.125g HC → 250ml PBS = 0.5g/L
0.1g HC → 100ml PBS = 1g/L
1g HC → 100ml PBS = 10g/L
9.34g HC → 100ml PBS = 93.4g/L

Results of test strip test

| | **Concentration of hydrocolloid in PBS** | | | | |
|---|---|---|---|---|---|
| **Test type** | **0.1g/L** | **0.5g/L** | **1g/L** | **10g/L** | **93.4g/L** |
| **Leukocytes (LEU)** | Neg | Neg | Neg | Neg | Neg |
| **Nitrite (NIT)** | Neg | Neg | Neg | Neg | Neg |
| **Urobilinogen (URO)** | Neg | Neg | Neg | Neg | Neg |
| **Protein (PRO)** | Neg | Neg | Neg | Neg | Neg |
| **pH** | 7 | 7 | 7 | 7 | 7 |
| **Blood (BLO)** | Neg | Neg | Neg | Neg | Neg |
| **Specific Gravity (SG)** | 1.015 | 1.015 | 1.015 | 1.015 | 1.01 |
| **Ketone (KET)** | Neg | Neg | Neg | Neg | Neg |
| **Bilirubin (BIL)** | Neg | Neg | Neg | Neg | Neg |
| **Glucose (GLU)** | Neg | Neg | Neg | Neg | Neg |

### Conclusions

No 'false' (positive) results were generated by exposing the test solutions to hydrocolloid.

The Welland hydrocolloid contains polymers. There was no detection of protein despite such a large amount of proteinaceous material

The hydrocolloid is not responsible for triggering any 'false positive' results in the Urine test strips used.

### Example 2

Testing a working prototype of a urinary tract infection diagnostic ostomy bag.

A diagnostic device was integrated into a urostomy pouch. A solution of simulated infected urine was formulated by addition of sodium nitrite and porcine esterase to Surine^{™} (a synthetic urine) used as the negative urine control. This well-known brand is widely used as a blank control in research and sport institutes.

The artificially infected urine was placed inside the test pouch to test if materials and components of the pouch system interfere with the diagnostic test result.

### Method

### Materials

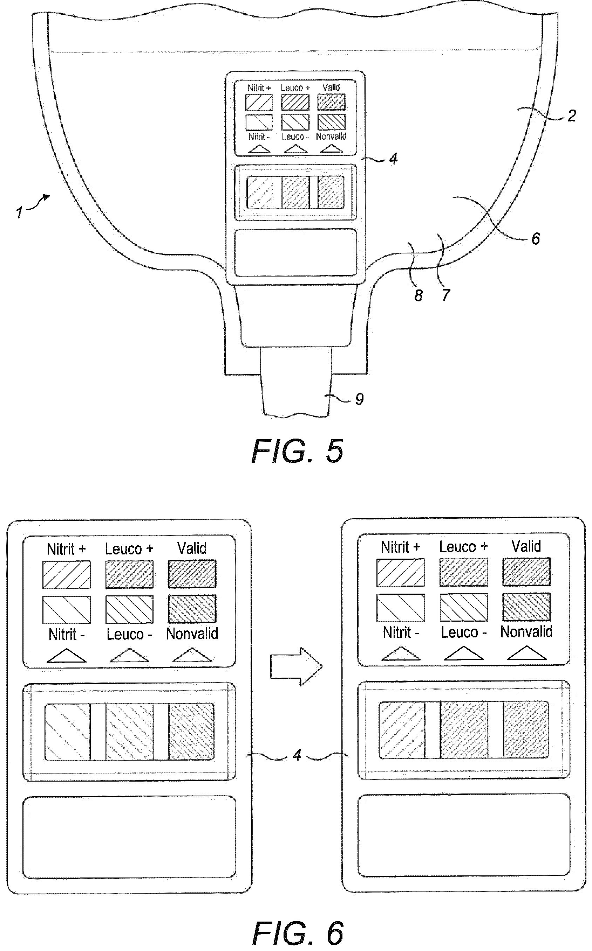

Diagnostic Device - (A) (shown in Figures 1, 2, 5 and 6)
Mission^{®} Urinalysis Strips - (B) (shown in Figure 4)
Sodium nitrite, puriss pa. ACS reagent ≥99%
Esterase from porcine liver, lyophilized powder ≥15 units/mg solid
Surine^{™} Negative urine control 1L
Urostomy pouch

Before assembling the prototype the infected urine simulant was created and demonstrated a positive result with the nitrite and leukocyte test. This was confirmed by testing the simulant with a 10 parameter urine test strip (B). The results are shown in Figure 4. The infected urine simulant was placed into the pouch with an integrated diagnostic device (A).

The infected urine was created by adding 3mg of sodium nitrite to 1000ml of Surine^{™}. This gave a concentration of 3mg/L of sodium nitrite. A solution of 100mg/L was also created to represent an advanced infection. The lower limit of detection for the detection of leukocytes is stated as a number of leukocytes per unit volume and not as an amount of esterase in solution i.e 10-20 leukocytes per micro-litre of urine. This made it difficult to calculate what quantity of esterase is appropriate to simulate a lower limit of detection, so, for this reason, the leukocyte esterase was weighed in at the same amount as the nitrite. A rough idea can be made for what 3mg/L of esterase, would represent in terms of leukocytes per unit volume because the mission test strips include a colour coded scale. The result for the strips shows a beige to light brown colour change for 20-70 Leu/µL. This suggests that 3mg/L would represent a very low concentration of leukocytes close to the limits stated on both products.

### Results

The results are shown in figures 4, 5 and 6.

### Discussion

The test strip leukocyte test produced a positive result more quickly with a colour change that was more discernible. The test strip specified a waiting time of 120 seconds.

The integrated diagnostic device used in the ostomy bag stated that the reading should be reconfirmed after 15 minutes since the valid indicator has appeared. This difference in speed is probably caused by the different chemicals used in the two devices.

### Conclusion

Devices A and B functioned as intended with a synthetic infected urine solution. Device A within the ostomy bag was not adversely influenced by the ostomy bag system, with regards to any influence on readings. Device B supported the readings of Device A with the synthetic infected urine solution.

Embodiments have been described herein in a concise way. It should be appreciated that features of these embodiments may be variously separated or combined within the invention. It will of course be understood that many variations may be made to the above-described embodiment without departing from the scope of the present invention.

## Claims

1. An ostomy bag (1) for an ostomate which comprises at least one pouch (2) wherein the pouch has an integrated diagnostic device (4) for detection of urinary tract infection and an inlet leading to a waste collection space defined by two opposing surfaces of two walls (7,8), the opposing surfaces configured to separate on the introduction of waste through the inlet (5) **characterized in that** the ostomy bag comprises a flange (3) adjacent to said at least one pouch (2), wherein the flange (3) adjoins a first wall of the pouch and the diagnostic device (4) adjoins a second wall (8) of the pouch (2) and, in use, wherein the diagnostic device (4) is provided in said second wall (8) facing away from the ostomate and wherein the diagnostic device (4) comprises a detection cell having an indicator panel having at least three indicators.

2. An ostomy bag (1) according to claim 1, wherein the ostomy bag (1) is a urostomy bag.

3. An ostomy bag (1) according to any one of the preceding claims, wherein the diagnostic device (4) is located substantially at or adjacent the bottom of the pouch (2) in use.

4. An ostomy bag (1) according to any one of the preceding claims, wherein the indicators are arranged adjacent to each other.

5. An ostomy bag (1) according to any one of the preceding claims, wherein a first indicator provides an optical signal when the detection cell comes into contact with fluid; a second indicator is capable of detecting and indicating the presence in urine of leukocytes or white blood cells; and a third indicator is capable of detecting and indicating the presence in urine of a nitrite.

6. An ostomy bag (1) according to any one of the preceding claims, wherein the diagnostic device (4) comprises a laminated structure having a number of layers.

7. An ostomy bag (1) according to claim 6, wherein an inner layer of the diagnostic device (4) comprises a planar member which defines an opening, through which any urine in the pouch (2) is able to pass and, in use, urine from an ostomate collects in the pouch (2) and enters the detection cell of the diagnostic device (4) via the opening.

8. An ostomy bag (1) according to claim 7, wherein a sheet is attached to the side of the planar member facing outwardly from the pouch (2) and a recess is provided over the opening in the planar member.

9. An ostomy bag (1) according to claim 8, wherein the recess contains a web of plastics material and on the web is provided the indicator panel of the detection cell.

10. An ostomy bag (1) according to claim 9, wherein the indicator panel comprises at least one indicator which comprises absorbent material and which provides an optical signal when a reagent on the absorbent material reacts with a specific analyte of interest.

11. An ostomy bag (1) according to any one of claims 6 to 10, wherein an outer layer of the detection cell is provided by a colourless film, which is impermeable to urine and forms the outer wall of the pouch (2) over the detection cell.

12. An ostomy bag (1) according to any one of the preceding claims, wherein the diagnostic device is welded or glued to the pouch (2).

13. An ostomy bag (1) according to any one of the preceding claims wherein the diagnostic device (4) is uncovered and visible externally, or the ostomy bag (1) comprises an outer pouch which covers the diagnostic device (4) and a window is provided in the outer pouch, allowing the detection cell to be viewed upon manipulation or movement of the outer pouch.

14. A pack of ostomy bags which includes at least one ostomy bag (1) according to any one of claims 1 to 9 together with a number of additional ostomy bags.

15. A method for producing an ostomy bag (1) according to any one of claims 1 to 9 which comprises the steps of providing an integrated diagnostic device (4) for detection of urinary tract infection in at least one wall of a pouch (2) of an ostomy bag (1).

## Patentansprüche

1. Stomabeutel (1) für einen Stomaträger, welcher mindestens eine Tasche (2) umfasst,
worin die Tasche eine integrierte Diagnosevorrichtung (4) zum Nachweis einer Harnwegsinfektion und einen Einlass aufweist, der zu einem Abfallauffangraum führt, der durch zwei gegenüberliegende Flächen zweier Wände (7, 8) begrenzt ist, worin die gegenüberliegenden Flächen ausgestaltet sind, sich beim Einleiten von Abfall durch den Einlass (5) zu trennen,
**dadurch gekennzeichnet, dass** der Stomabeutel einen Flansch (3) umfasst, der an die mindestens eine Tasche (2) angrenzt,
worin der Flansch (3) an eine erste Wand (7) der Tasche angrenzt und die Diagnosevorrichtung (4) an eine zweite Wand (8) der Tasche (2) angrenzt, und worin bei der Verwendung die Diagnosevorrichtung (4) in der zweiten Wand (8) vorgesehen ist, die vom Stomaträger abgewandt ist, und worin die Diagnosevorrichtung (4) eine Detektionszelle mit einer Anzeigefläche mit mindestens drei Anzeigen umfasst.

2. Stomabeutel (1) gemäß Anspruch 1, worin der Stomabeutel (1) ein Urostomiebeutel ist.

3. Stomabeutel (1) gemäß einem der vorstehenden Ansprüche, worin sich die Diagnosevorrichtung (4) bei der Verwendung im Wesentlichen am oder neben dem Boden der Tasche (2) befindet.

4. Stomabeutel (1) gemäß einem der vorstehenden Ansprüche, worin die Indikatoren nebeneinander angeordnet sind.

5. Stomabeutel (1) gemäß einem der vorstehenden Ansprüche, worin ein erster Indikator ein optisches Signal liefert, wenn die Detektionszelle mit Flüssigkeit in Kontakt kommt; ein zweiter Indikator geeignet ist, das Vorhandensein von Leukozyten oder weißen Blutkörperchen im Urin zu erkennen und anzuzeigen; und ein dritter Indikator geeignet ist, das Vorhandensein von Nitrit im Urin zu erkennen und anzuzeigen.

6. Stomabeutel (1) gemäß einem der vorstehenden Ansprüche, worin die Diagnosevorrichtung (4) eine laminierte Struktur mit einer Anzahl von Schichten umfasst.

7. Stomabeutel (1) gemäß Anspruch 6, worin eine innere Schicht der Diagnosevorrichtung (4) ein flaches Element umfasst, das eine Öffnung definiert, durch die jeglicher Urin in der Tasche (2) hindurchtreten kann, und worin sich bei der Verwendung Urin von einem Stomaträger in der Tasche (2) sammelt und über die Öffnung in die Detektionszelle der Diagnosevorrichtung (4) gelangt.

8. Stomabeutel (1) gemäß Anspruch 7, worin eine Folie an der Seite des flachen Elements angebracht ist, die von der Tasche (2) nach außen zeigt, und eine Aussparung über der Öffnung im flachen Element vorgesehen ist.

9. Stomabeutel (1) gemäß Anspruch 8, worin die Aussparung ein Netz aus Kunststoffmaterial enthält und auf dem Netz die Anzeigefläche der Detektionszelle vorgesehen ist.

10. Stomabeutel (1) gemäß Anspruch 9, worin die Anzeigefläche mindestens einen Indikator umfasst, der absorbierendes Material umfasst und ein optisches Signal liefert, wenn ein Reagenz auf dem absorbierenden Material mit einem bestimmten Analyten von Interesse reagiert.

11. Stomabeutel (1) gemäß einem der Ansprüche 6 bis 10, worin eine äußere Schicht der Detektionszelle durch eine farblose Folie gebildet wird, die für Urin undurchlässig ist und die Außenwand der Tasche (2) über der Detektionszelle bildet.

12. Stomabeutel (1) gemäß einem der vorstehenden Ansprüche, worin die Diagnosevorrichtung mit der Tasche (2) verschweißt oder verklebt ist.

13. Stomabeutel (1) gemäß einem der vorstehenden Ansprüche, worin die Diagnosevorrichtung (4) unbedeckt und von außen sichtbar ist oder der Stomabeutel (1) eine Außentasche umfasst, die die Diagnosevorrichtung (4) bedeckt, und worin in der Außentasche ein Fenster vorgesehen ist, durch das die Detektionszelle bei Manipulation oder Bewegung der Außentasche sichtbar ist.

14. Packung mit Stomabeuteln, welche mindestens einen Stomabeutel (1) gemäß einem der Ansprüche 1 bis 9 zusammen mit einer Anzahl zusätzlicher Stomabeutel enthält.

15. Verfahren zur Herstellung eines Stomabeutels (1) gemäß einem der Ansprüche 1 bis 9, welches die Schritte umfasst, Bereitstellen einer integrierten Diagnosevorrichtung (4) zur Erkennung einer Harnwegsinfektion in mindestens einer Wand einer Tasche (2) eines Stomabeutels (1).

## Revendications

1. Poche de stomie (1) pour une personne stomisée qui comprend au moins une poche (2), dans laquelle la poche présente un dispositif de diagnostic (4) intégré pour la détection d'infections urinaires et une entrée menant à un espace de collecte de déchets défini par deux surfaces opposées de deux parois (7, 8),
les surfaces opposées étant configurées pour se séparer lors de l'introduction de déchets par l'entrée (5), **caractérisée en ce que** la poche de stomie comprend une bride (3) adjacente à ladite au moins une poche (2), dans laquelle la bride (3) est adjacente à une première paroi (7) de la poche et le dispositif de diagnostic (4) est adjacent à une deuxième paroi (8) de la poche (2) et, pendant l'utilisation, dans laquelle le dispositif de diagnostic (4) est disposé dans ladite deuxième paroi (8) orientée à l'opposé de la personne stomisée et dans laquelle le dispositif de diagnostic (4) comprend une cellule de détection présentant un panneau d'indicateurs ayant au moins trois indicateurs.

2. Poche de stomie (1) selon la revendication 1, dans laquelle la poche de stomie (1) est une poche d'urostomie.

3. Poche de stomie (1) selon l'une quelconque des revendications précédentes, dans laquelle le dispositif de diagnostic (4) est situé sensiblement au niveau ou à proximité du fond de la poche (2) pendant l'utilisation.

4. Poche de stomie (1) selon l'une quelconque des revendications précédentes, dans laquelle les indicateurs sont agencés les uns à côté des autres.

5. Poche de stomie (1) selon l'une quelconque des revendications précédentes, dans laquelle un premier indicateur fournit un signal optique lorsque la cellule de détection entre en contact avec un fluide ; un deuxième indicateur est capable de détecter et d'indiquer la présence de leucocytes ou de globules blancs dans l'urine ; et un troisième indicateur est capable de détecter et d'indiquer la présence d'un nitrite dans l'urine.

6. Poche de stomie (1) selon l'une quelconque des revendications précédentes, dans laquelle le dispositif de diagnostic (4) comprend une structure stratifiée présentant un certain nombre de couches.

7. Poche de stomie (1) selon la revendication 6, dans laquelle une couche interne du dispositif de diagnostic (4) comprend un élément plan qui définit une ouverture, par laquelle toute urine dans la poche (2) peut s'écouler et, pendant l'utilisation, l'urine d'une personne stomisée s'accumule dans la poche (2) et pénètre dans la cellule de détection du dispositif de diagnostic (4) par l'ouverture.

8. Poche de stomie (1) selon la revendication 7, dans laquelle une feuille est fixée sur le côté de l'élément plan orienté vers l'extérieur de la poche (2) et un évidement est disposé sur l'ouverture dans l'élément plan.

9. Poche de stomie (1) selon la revendication 8, dans laquelle l'évidement contient une bande de matière plastique et le panneau d'indicateurs de la cellule de détection est disposé sur la bande.

10. Poche de stomie (1) selon la revendication 9, dans laquelle le panneau d'indicateurs comprend au moins un indicateur qui comprend une matière absorbante et qui fournit un signal optique lorsqu'un réactif présent sur la matière absorbante réagit avec un analyte spécifique d'intérêt.

11. Poche de stomie (1) selon l'une quelconque des revendications 6 à 10, dans laquelle une couche externe de la cellule de détection est constituée d'un film incolore, qui est imperméable à l'urine et forme la paroi externe de la poche (2) sur la cellule de détection.

12. Poche de stomie (1) selon l'une quelconque des revendications précédentes, dans laquelle le dispositif de diagnostic est soudé ou collé à la poche (2).

13. Poche de stomie (1) selon l'une quelconque des revendications précédentes, dans laquelle le dispositif de diagnostic (4) n'est pas couvert et est visible de l'extérieur, ou la poche de stomie (1) comprend une poche externe qui recouvre le dispositif de diagnostic (4) et une fenêtre est ménagée dans la poche externe, ce qui permet de voir la cellule de détection lors de la manipulation ou du déplacement de la poche externe.

14. Ensemble de poches de stomie qui inclut au moins une poche de stomie (1) selon l'une quelconque des revendications 1 à 9, ainsi qu'un certain nombre de poches de stomie supplémentaires.

15. Procédé de fabrication d'une poche de stomie (1) selon l'une quelconque des revendications 1 à 9
qui comprend les étapes de fourniture d'un dispositif de diagnostic (4) intégré pour la détection d'une infection urinaire dans au moins une paroi d'une poche (2) d'une poche de stomie (1).
